# EUROPEAN PATENT APPLICATION

(11) **EP 1 406 127 A2**
(43) Date of publication of application: **07.04.2004**
(21) Application number: 03255986.6
(22) Date of filing: 23.09.2003
(51) Int. Cl.: G03G 5/06, C07D 209/86, C07C 211/48

(54) **Organophotoreceptor with a compound having a toluidine group**

(30) Priority: 30.09.2002 US 414822 P
(71) Applicant: SAMSUNG ELECTRONICS CO., LTD., Suwon-City, Kyungki-do (KR)
(72) Inventor: Jubran, Nusrallah, St. Paul, MN 55119 (US); Law, Kam W., Woodbury, MN 55125 (US); Tokarski, Zbigniew, Woodbury, MN 55125 (US)
(74) Representative: Moy, David

(57) **Abstract**

An improved organophotoreceptor comprises an electrically conductive substrate and a photoconductive element on the electrically conductive substrate wherein the photoconductive element comprises
a) a charge transport material with the following formula 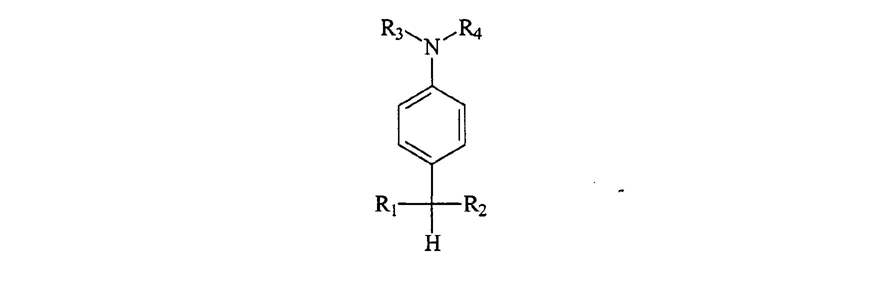 where R₁ and R₂ are, independently, a carbazolyl group, an (N,N-disubstituted) aminoaryl group, such as a triphenyl amine group, or a julolidine group, and R₃ and R₄ are, independently, hydrogen, branched or linear alkyl group (e.g., a C₁-C₂₀ alkyl group), branched or linear unsaturated hydrocarbon group, cycloalkyl group (e.g. cyclohexyl group), or aryl group (e.g., phenyl group, naphthyl group, stilbenyl group, (9H-fluoren-9-ylidene)benzyl group, or tolanyl group);
(b) an optional charge transport compound; and
(c) a charge generating compound.

## Description

This application claims priority to copending U.S. Provisional Application serial number 60/414,822 filed on September 30, 2002 to Jubran et al., entitled "Organophotoreceptors With Novel Electron Transport Compound Having A Toluidine Group," incorporated herein by reference.

This invention relates to organophotoreceptors suitable for use in electrophotography and, more specifically, to organophotoreceptors having an electron transport compound comprising a toluidine group, which may be substituted.

In electrophotography, an organophotoreceptor in the form of a plate, disk, sheet, belt, drum or the like having an electrically insulating photoconductive element on an electrically conductive substrate is imaged by first uniformly electrostatically charging the surface of a photoconductive element, and then exposing the charged surface to a pattern of light. The light exposure selectively dissipates the charge in the illuminated areas where light strikes the surface, thereby forming a pattern of charged and uncharged areas referred to as a latent image. A liquid toner or solid toner can then be provided in the vicinity of the latent image, and toner droplets or particles can be deposited in either the charged or uncharged areas depending on the properties of the toner to create a toned image on the surface of the photoconductive element. The resulting toned image can be transferred to a suitable ultimate or intermediate receiving surface, such as paper, or the photoconductive element can operate as the ultimate receptor for the image. The imaging process can be repeated many times to complete a single image, which can involve, for example, overlying images of distinct color components or effecting shadow images to complete a full color complete image, and/or to reproduce additional images.

Both single layer and multilayer photoconductive elements have been used. In the single layer embodiment, charge generating compound and a charge transport material selected from the group consisting of a charge transport compound, an electron transport compound, and a combination of both are combined with a polymeric binder and then deposited on the electrically conductive substrate. In the multilayer embodiments based on a charge transport compound, the charge transport compound and charge generating compound are in the form of separate layers, each of which can optionally be combined with a polymeric binder, deposited on the electrically conductive substrate. Two arrangements are possible. In one arrangement (the "dual layer" arrangement), the charge generating layer is deposited on the electrically conductive substrate and the charge transport layer is deposited on top of the charge generating layer. In an alternate arrangement (the "inverted dual layer" arrangement), the order of the charge transport layer and charge generating layer is reversed.

In both the single and multilayer photoconductive elements, the purpose of the charge generating material is to generate charge carriers (i.e., holes and/or electrons) upon exposure to light. The purpose of the charge transport material is to accept these charge carriers and transport them through the charge transport layer in order to discharge a surface charge on the photoconductive element. When a charge transport compound is used, the charge transport compound accepts the hole carriers and transports them through the layer in which the charge transport compound is located. When an electron transport compound is used, the electron transport compound accepts the electron carriers and transports them through the layer in which the electron transport compound is located.

According to the present invention there is provided an organophotoreceptor, an electrophotographic imaging apparatus, an electrophotographic imaging process, and a charge transport material, as set forth in the appended claims.

Preferred features of the invention will be apparent from the dependent claims, and the description which follows.

This invention pertains to organophotoreceptors having good electrostatic properties such as high V_{acc} and low V_{dis}.

In a first aspect, the invention features an organophotoreceptor that comprises an electrically conductive substrate and a photoconductive element on the electrically conductive substrate wherein the photoconductive element comprises
a) a charge transport material having the following formula 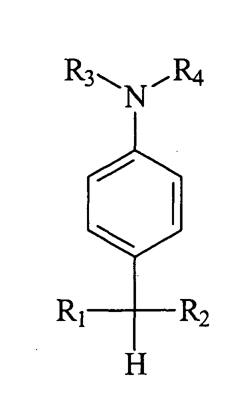 where R₁ and R₂ are, independently, carbazolyl group, an (N,N-disubstituted) aminoaryl group, such as a triphenylamine group, or julolidine group, and R₃ and R₄ are, independently, hydrogen, branched or linear alkyl group (e.g., a C₁-C₂₀ alkyl group), branched or linear unsaturated hydrocarbon group, cycloalkyl group (e.g. cyclohexyl group), or aryl group (e.g., phenyl group, naphthyl group, stilbenyl group, (9H-fluoren-9-ylidene)benzyl group, or tolanyl group);
(b) an optional charge transport compound; and
(c) a charge generating compound.

The organophotoreceptor may be provided, for example, in the form of a plate, a flexible belt, a flexible disk, a sheet, a rigid drum, or a sheet around a rigid or compliant drum. In one embodiment, the organophotoreceptor includes: (a) a photoconductive element comprising the charge transport compound, the charge generating compound, the electron transport compound, and a polymeric binder; and (b) the electrically conductive substrate.

In a second aspect, the invention features an electrophotographic imaging apparatus that comprises (a) a light imaging component; and (b) the above described organophotoreceptor oriented to receive light from the light imaging component. The apparatus can further include a toner dispenser, such as a liquid toner dispenser. The method of electrophotographic imaging with photoreceptors containing these improved transport materials is also described.

In a third aspect, the invention features an electrophotographic imaging process comprising (a) applying an electrical charge to a surface of the above-described organophotoreceptor; (b) imagewise exposing the surface of the organophotoreceptor to radiation to dissipate charge in selected areas and thereby form a pattern of at least relatively charged and uncharged areas on the surface; (c) contacting the surface with a toner, such as a liquid toner that includes a dispersion of colorant particles in an organic liquid, to create a toned image; and (d) transferring the toned image to a substrate.

In a fourth aspect, the invention features a charge transport material having the general formula above.

The invention provides charge transport materials for organophotoreceptors featuring a combination of good mechanical and electrostatic properties. These photoreceptors can be used successfully with liquid toners to produce high quality images. The high quality of the imaging system is maintained after repeated cycling.

Other features and advantages of the invention will be apparent from the following description of the preferred embodiments thereof, and from the claims.

Fig. 1 is a schematic side view of an organophotoreceptor with a photoconductive layer on an electrically conductive substrate.

Fig. 2 is a schematic side view of an organophotoreceptor with a charge generating layer and a charge transport layer sequentially on an electrically conductive substrate.

Fig. 3 is a schematic side view of an organophotoreceptor with a charge transport layer and a charge generating layer sequentially on an electrically conductive substrate.

Fig. 4 is a schematic side view of an organophotoreceptor with a charge transport layer, a charge generating layer and an electron transport layer sequentially on an electrically conductive substrate.

Fig. 5 is a schematic side view of an organophotoreceptor with an electron transport layer, a charge generating layer and a charge transport layer sequentially on an electrically conductive substrate.

Improved organophotoreceptors comprise a compound generally effective as a charge transport material having the formula: 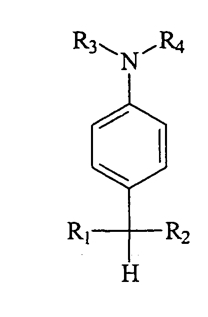 where R₁ and R₂ are, independently, an (N,N-disubstituted) aminoaryl group (such as a triphenylamine group), a carbazolyl group, or a julolidine group, and R₃ and R₄ are, independently, hydrogen, branched or linear alkyl group (e.g., a C₁-C₂₀ alkyl group), branched or linear unsaturated hydrocarbon group, cycloalkyl group (e.g. cyclohexyl group), or aryl group (e.g., phenyl group, naphthyl group, stilbenyl group, (9H-fluoren-9-ylidene)benzyl group, or tolanyl group). Substitution is liberally allowed on the groups to effect various physical effects on the properties of the compounds, such as mobility, sensitivity, solubility, stability, and the like, as is known in the art. Compounds described by the above formula also include isomeric equivalencies of the formula above, meaning that R₁ and R₂ are interchangeable and R₃ and R₄ are interchangeable. In some embodiments, these compounds are particularly effective as electron transport compounds.

In general, the organophotoreceptor comprises an electrically conductive substrate having a photoconductive element on a surface of the electrically conductive substrate in which the photoconductive element can include one or more layers, i.e., sublayers, within its structure. One or more of the layers of the photoconductor may include an electron transport compound. A layer with the electron transport compound can also comprise a polymeric binder, a charge transport compound, a UV light stabilizer, and/or a charge generating compound.

To produce high quality images, particularly after multiple cycles, it is desirable for the compounds of the organophotoreceptor to form a homogeneous solution with the polymeric binder and remain approximately homogeneously distributed through the organophotoreceptor material during the cycling of the material. In addition, to produce high quality images, it is desirable to increase the amount of charge that the organophotoreceptor can accept (indicated by a parameter known as the acceptance voltage or "V_{acc}"), and to reduce retention of that charge upon discharge (indicated by a parameter known as the discharge voltage or "V_{dis}").

The improved organophotoreceptors described herein can have a high V_{acc}, a low V_{dis}, and high stabilities with respect to cycling testing, crystallization, bending and stretching. The organophotoreceptors are particularly useful in laser printers and the like as well as photocopiers, scanners, other electronic devices based on electrophotography, and a combination thereof. The use of these organophotoreceptors is described in more detail below in the context of laser printer use, although their application in other devices operating by electrophotography can be generalized from the discussion below.

Electron transport compounds, in general, have an appropriate ability to transport electrons, in contrast with charge transport compounds, which are generally more effective at transporting holes, i.e., positive charges. In electrophotography applications, a charge generating compound within an organophotoreceptor absorbs light to form electron-hole pairs. The electron and/or hole can be transported over an appropriate time frame under a large electric field to discharge locally a surface charge that is generating the field. The discharge of the field at a particular location results in a surface charge pattern that essentially matches the pattern drawn with the light. This charge pattern then can be used to guide toner deposition. To print a two dimensional image using the organophotoreceptor, the organophotoreceptor has a two dimensional surface for forming at least a portion of the image. The imaging process then continues by cycling the organophotoreceptor to complete the formation of the entire image and/or for the processing of subsequent images.

The organophotoreceptor may be provided in the form of a plate, a flexible belt, a disk, a rigid drum, a sheet around a rigid or compliant drum, or the like. The charge transport compound and/or the electron transport compound can be in the same layer as the charge generating compound and/or in a different layer from the charge generating compound. For example, the electron transport compound may be in an overcoat layer. In some embodiments, the organophotoreceptor material has a single layer with both a charge transport compound and a charge generating compound within a polymeric binder. In further embodiments, a charge transport compound can be in a charge transport layer distinct from the charge generating layer. For embodiments with an electron transport layer and a separate charge transport layer, a charge generating layer generally is intermediate between the charge transport layer and the electron transport layer. For embodiments with an electron transport layer in an overcoat layer, a charge transport layer may be intermediate between the charge generating layer and the electrically conductive substrate. Additional layers can be used also, as described further below.

The organophotoreceptors can be incorporated into an electrophotographic imaging apparatus, such as laser printers. In these devices, an image is formed from physical embodiments and converted to a light image that is scanned onto the organophotoreceptor to form a surface latent image. The surface latent image can be used to attract toner onto the surface of the organophotoreceptor, in which the toner image is the same or the negative of the light image projected onto the organophotoreceptor. The toner can be a liquid toner or a dry toner. The toner can be subsequently transferred, from the surface of the organophotoreceptor, to a receiving surface, such as a sheet of paper. After the transfer of the toner, the entire surface can be discharged, and the material is ready to cycle again. The imaging apparatus can further comprise, for example, a plurality of support rollers for transporting a paper receiving medium and/or for movement of the photoreceptor, a light imaging component with suitable optics to form the light image, a light source, such as a laser, a toner source and delivery system and an appropriate control system.

An electrophotographic imaging process generally can comprise (a) applying an electrical charge to a surface of the above-described organophotoreceptor; (b) imagewise exposing the surface of the organophotoreceptor to radiation to dissipate charge in selected areas and thereby form a pattern of charged and uncharged areas on the surface; (c) exposing the surface with a toner, such as a liquid toner that includes a dispersion of colorant particles in an organic liquid to create a toner image, to attract toner to the charged or discharged regions of the organophotoreceptor; and (d) transferring the toner image to a substrate.

In describing chemicals by structural formulae with chemical substituents, certain terms can be used in association with a formula to reflect the full scope represented by the formula. For example, the terms "group," "moiety," and "derivatives" can have particular meanings. The term "group" indicates that the generically recited chemical material (e.g., alkyl group, phenyl group, fluorenylidene malonitrile group, carbazole hydrazone group, etc.) may have any substituent thereon which is consistent with the bond structure of that group. Thus, the term 'group' allows for the presence of further substitution on the named class of materials, as long as the substitutent is still recognizable as within the generic class. For example, alkyl group includes, for example, unsubstituted liner, branched and cyclic alkyls, such as methyl, ethyl, isopropyl, tert-butyl, cyclohexyl, dodecyl and the like, and also includes such substituted alkyls such as chloromethyl, dibromoethyl, 1,3-dicyanopropyl, 1,3,5-trihydroxyhexyl, 1,3,5-trifluorocyclohexyl, 1-methoxy-dodecyl, phenylpropyl and the like. However, as is consistent with such nomenclature, no substitution would be included within the term that would alter the fundamental bond structure of the underlying group. For example, where a phenyl ring group_ is recited, substitution such as 1-hydroxyphenyl, 2,4-fluorophenyl, orthocyanophenyl, 1,3,5-trimethoxyphenyl and the like would be acceptable within the terminology, while substitution of 1,1,2,2,3,3-hexamethylphenyl would not be acceptable as that substitution would require the ring bond structure of the phenyl group to be altered to a non-aromatic form because of the substitution. Similarly, when referring to carbazolyl group, the compound or substitutent cited includes any substitution that does not substantively alter the chemical nature of the ring groups or other salient bond structures in the formula (e.g., double bonds between nitrogens, etc.). For example, carbazolyl group would not include such alteration of the ring wherein aromaticity is removed by saturation of double bonds in the ring, while the addition of a long chain fatty acid group to replace a hydrogen atom on the carbazolyl group would be included.

Where the term moiety is used, such as alkyl moiety or phenyl moiety, that terminology indicates that the chemical material is not substituted. For example, the term alkyl moiety represents only an unsubstituted alkyl hydrocarbon group, whether branched, straight chain, or cyclic. Where the term derivative is used, that terminology indicates that a compound is derived or obtained from another and containing essential elements of the parent substance.

There are many electron transport compounds available for electrophotography. Some more common electron transport compounds in the art are enumerated below. Generally, these electron transport compounds suffers some disadvantages in at least one of the various electrophotographic applications. Therefore, there is always a need for additional electron transport compounds to meet the various requirements of electrophotographic applications. Some embodiments of the compounds described herein with a toluidine group can be used to meet some of these needs.

### Organophotoreceptors

The organophotoreceptor may be, for example, in the form of a plate, a sheet, a flexible belt, a disk, a rigid drum, or a sheet around a rigid or compliant drum, with flexible belts and rigid drums generally being used in commercial embodiments. The organophotoreceptor may comprise, for example, an electrically conductive substrate and a photoconductive element in-the form of one or more layers. The organophotoreceptor generally comprises both a charge transport compound and a charge generating compound in a polymeric binder, which may or may not be in the same layer. Similarly, an electron transport compound may or may not be in the same layer with the charge generating compound. If the electron transport compound is in a different layer from the charge generating compound, the electron transport compound can be an overcoat, i.e., on the side opposite the electrically conductive substrate, or an undercoat, on the same side of the charge generating layer as the electrically conductive substrate. In some embodiments, a layer with an electron transport compound can further include an ultraviolet light stabilizer.

With respect to the charge generation compound and the charge transport compound, in some embodiments with a single layer construction, the charge transport compound and the charge generating compound are in a single layer. In other embodiments, however, the photoconductive element comprises a bilayer construction featuring a charge generating layer and a separate charge transport layer. The charge generating layer may be located intermediate between the electrically conductive substrate and the charge transport layer. Alternatively, the photoconductive element may have a structure in which the charge transport layer is intermediate between the electrically conductive substrate and the charge generating layer. Comparable organophotoreceptors can be formed with only an electron transport compound without a charge transport compound, although embodiments of organophotoreceptors of particular interest have a charge transport compound.

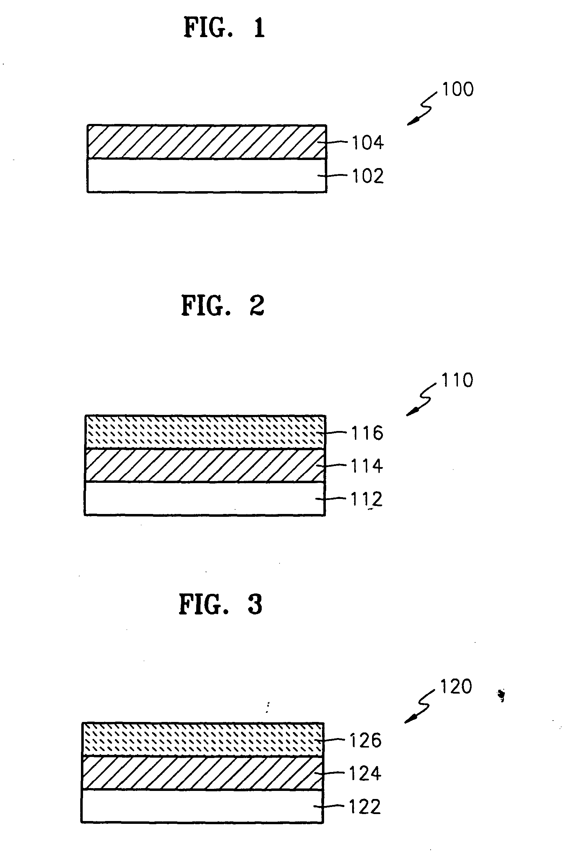

Based on the three basic structures of the charge generating layer and the charge transport layer, the structure of the organophotoreceptor can be generalized to account for the presence of an electron transport compound. For example, for embodiments in which the electron transport compound is in the same layer as the charge generating compound, there are three possible structures shown schematically in Figs. 1-3. Referring to Fig. 1, organophotoreceptor 100 comprises an electrically conductive substrate 102 and a photoconductive layer 104 comprising a charge generating compound, a charge transport compound and an electron transport compound. Referring to Fig. 2, organophotoreceptor 110 comprises an electrically conductive substrate 112, a charge generating layer 114 comprising a charge generating compound and an electron transport compound, and charge transport layer 116 comprising a charge transport compound. Referring to Fig. 3, organophotoreceptor 120 comprises an electrically conductive substrate 122, a charge transport layer 124 comprising a charge transport compound and a charge generating layer 126 comprising a charge generating compound and an electron transport compound.

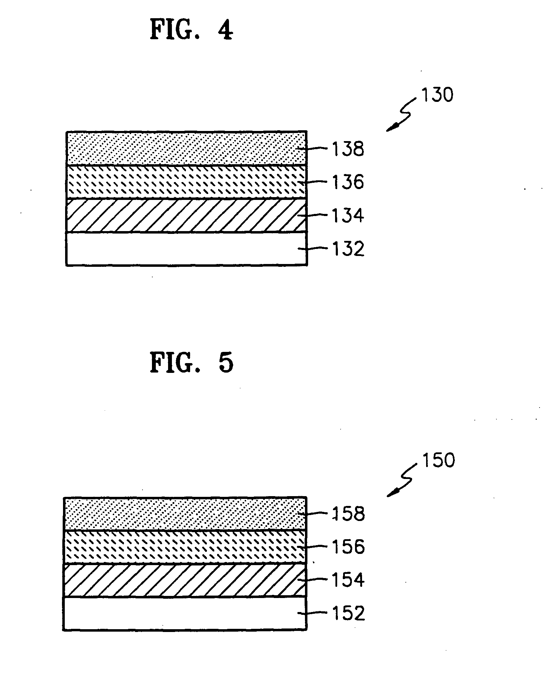

For embodiments in which the electron transport compound is in a different layer than the charge generating compound, there are two structures of primary interest, which are shown in Figs. 4 and 5. Referring to Fig. 4, organophotoreceptor 130 comprises an electrically conductive substrate 132, a charge transport layer 134 comprising a charge transport compound, a charge generating layer 136 comprising a charge generating compound, and an electron transport layer 138 comprising an electron transport compound. Referring to Fig. 5, organophotoreceptor 150 comprises an electrically conductive substrate 152, an electron transport layer 154 comprising an electron transport compound, a charge generating layer 156 comprising a charge generating compound, and a charge transport layer 158 comprising a charge transport compound.

While the embodiments of Figs. 1-5 have electron transport compound in a single layer, multiple layers can comprise an electron transport compound. In particular, an electron transport layer and a charge generating layer can both comprise an electron transport compound. Furthermore, the organophotoreceptor structures shown in Figs. 1-5 can further comprise additional undercoat and/or overcoat layers such as those described further below. In addition, other layered organophotoreceptor structures can be formed beyond the embodiments of particular interest shown in Figs. 1-5, and these additional structures can have different layer ordering and/or multiple layers of the types described with or without different compositions.

The electrically conductive substrate, along with an electrically insulating substrate supporting the electrically conductive substrate, may be flexible, for example in the form of a flexible web or a belt, or inflexible, for example in the form of a drum. A drum can have a hollow cylindrical structure that provides for attachment of the drum to a drive that rotates the drum during the imaging process. Typically, the combined substrate comprises an electrically insulating substrate and a thin layer of electrically conductive material as the electrically conductive substrate onto which the photoconductive material is applied.

The electrically insulating substrate may be paper or a film forming polymer such as a polyester, e.g., polyethylene terephthalate or polyethylene naphthalate, a polyimide, a polysulfone, a polyolefin, e.g., polyethylene or polypropylene, a nylon, a polycarbonate, a polyvinyl resin, a polyvinyl fluoride, a polystyrene, suitable copolymers thereof, mixtures thereof and the like. Specific examples of polymers for supporting substrates include, for example, polyethersulfone (Stabar^{TM} S-100, available from ICI), polyvinyl fluoride (Tedlar®, available from E.I. DuPont de Nemours & Company), polybisphenol-A polycarbonate (Makrofol™, available from Mobay Chemical Company) and amorphous polyethylene terephthalate (Melinar^{TM}, available from ICI Americas, Inc.). The electrically conductive materials, generally in the form of a coated layer, may comprise graphite, dispersed carbon black, iodide, conductive polymers, such as polypyroles and Calgon® conductive polymer 261 (commercially available from Calgon Corporation, Inc., Pittsburgh, Pa.), metals, such as aluminum, titanium, chromium, brass, gold, copper, palladium, nickel, stainless steel or alloys thereof, a metal oxide such as tin oxide or indium oxide, or combinations thereof. In embodiments of particular interest, the electrically conductive material comprises aluminum metal. Generally, the photoconductor substrate has a thickness adequate to provide the required mechanical stability. For example, flexible web substrates generally have a thickness from about 0.01 to about 1 mm, while drum substrates generally have a thickness from about 0.5 mm to about 2 mm.

The charge generating compound generally is a material, such as a dye or pigment, which is capable of absorbing light to generate charge carriers. Non-limiting examples of suitable charge generating compounds include, for example, metal-free phthalocyanines (e.g., CGM-X01 available from Sanyo Color Works, Ltd.), metal phthalocyanines such as titanium phthalocyanine, copper phthalocyanine, oxytitanium phthalocyanine (also referred to as titanyl oxyphthalocyanine, and including any crystalline phase or mixtures of crystalline phases that can act as a charge generating compound), hydroxygallium phthalocyanine, squarylium dyes and pigments, hydroxy-substituted squarylium pigments, perylimides, polynuclear quinones available from Allied Chemical Corporation under the trade name Indofast® Double Scarlet, Indofast® Violet Lake B, Indofast® Brilliant Scarlet and Indofast® Orange, quinacridones available from DuPont under the trade name Monastral™ Red, Monastral^{TM} Violet and Monastral™ Red Y, naphthalene 1,4,5,8-tetracarboxylic acid derived pigments including the perinones, tetrabenzoporphyrins and tetranaphthaloporphyrins, indigo- and thioindigo dyes, benzothioxanthene-derivatives, perylene 3,4,9,10-tetracarboxylic acid derived pigments, polyazo-pigments including bisazo-, trisazo- and tetrakisazo-pigments, polymethine dyes, dyes containing quinazoline groups, tertiary amines, amorphous selenium, selenium alloys such as selenium-tellurium, selenium-tellurium-arsenic and selenium-arsenic, cadmium sulphoselenide, cadmium selenide, cadmium sulphide, and mixtures thereof. For some embodiments, the charge generating compound comprises oxytitanium phthalocyanine (e.g., any phase thereof), hydroxygallium phthalocyanine or a combination thereof.

There are many kinds of charge transport compounds available for electrophotography. For example, any charge transport compound known in the art can be used to form organophotoconductors described herein. Suitable charge transport compounds include, but are not limited to, pyrazoline derivatives, fluorene derivatives, oxadiazole derivatives, stilbene derivatives, hydrazone derivatives, carbazole hydrazone derivatives, triaryl amines, polyvinyl carbazole, polyvinyl pyrene, polyacenaphthylene, or multi-hydrazone compounds comprising at least two hydrazone groups and at least two groups selected from the group consisting of triphenylamine and heterocycles such as carbazole, julolidine, phenothiazine, phenazine, phenoxazine, phenoxathiin, thiazole, oxazole, isoxazole, dibenzo (1,4) dioxine, thianthrene, imidazole, benzothiazole, benzotriazole, benzoxazole, benzimidazole, quinoline, isoquinoline, quinoxaline, indole, indazole, pyrrole, purine, pyridine, pyridazine, pyrimidine, pyrazine, triazole, oxadiazole, tetrazole, thiadiazole, benzisoxazole, benzisothiazole, dibenzofuran, dibenzothiophene, thiophene, thianaphthene, quinazoline, cinnoline or combinations thereof. In some embodiments, the charge transport compound is a enamine stilbene compound such as MPCT-10, MPCT -38, and MPCT-46 from Mitsubishi Paper Mills (Tokyo, Japan).

A suitable electron transport composition for use in the appropriate layer or layers generally has an electron affinity that is large relative to potential electron traps while yielding an appropriate electron mobility in a composite with a polymer. In some embodiments, the electron transport composition has a reduction potential less than O₂. In general, electron transport compositions are easy to reduce and difficult to oxidize while charge transport compositions generally are easy to oxidize and difficult to reduce. In some embodiments, the electron transport compounds have a room temperature, zero field electron mobility of at least about 1 x 10⁻¹³ cm²/Vs, in further embodiments at least about 1 x 10⁻¹⁰ cm²/Vs, in additional embodiments at least about 1 x 10⁻⁸ cm²/Vs, and in other embodiments at least about 1 x 10⁻⁶ cm²/Vs. A person of ordinary skill in the art will recognize that other ranges of electron mobility within the explicit ranges are contemplated and are within the present disclosure.

Electron transport compounds for use in organophotoreceptors can be suitable embodiments of compounds with a toluidine group, i.e., a toluidine-based compound, as described further below. One or more toluidine-based compounds can be used in a selected layer of the photoconductor as an electron transport compound(s), or the toluidine-based compound(s) can be used in combination with another non-toluidine-based electron transport compound. Non-limiting examples of suitable non-toluidine-based electron transport compounds include, for example, bromoaniline, tetracyanoethylene, tetracyanoquinodimethane, 2,4,7-trinitro-9-fluorenone, 2,4,5,7-tetranitro-9-fluorenone, 2,4,5,7-tetranitroxanthone, 2,4,8-trinitrothioxanthone, 2,6,8-trinitro-indeno4H-indeno [1,2-b] thiophene-4-one, and 1,3,7-trinitrodibenzothiophene-5,5-dioxide, (2,3-diphenyl-1-indenylidene)malononitrile, 4H-thiopyran-1,1-dioxide and its derivatives, such as 4-dicyanomethylene-2,6-diphenyl-4H-thiopyran-1,1-dioxide, 4-dicyanomethylene-2,6-di-m-tolyl-4H-thiopyran-1,1-dioxide, and unsymmetrically substituted 2,6-diaryl-4H-thiopyran-1,1-dioxide such as 4H-1,1-dioxo-2-(p-isopropyl phenyl)-6-phenyl-4-(dicyanomethylidene)thiopyran and 4H-1,1-dioxo-2-(p-isopropyl phenyl)-6-(2-thienyl)-4-(dicyanomethylidene)thiopyran, derivatives of phospha-2,5-cyclohexadiene, alkoxycarbonyl 9-fluorenylidene)malononitrile derivatives such as (4-n-butoxycarbonyl-9-fluorenylidene)malononitrile, (4-phenethoxycarbonyl-9-fluorenylidene) malononitrile, (4-carbitoxy-9-fluorenylidene)malononitrile, and diethyl(4-n-butoxy carbonyl-2,7-dinitro-9-fluorenylidene)-malonate, anthraquino dimethane derivatives such as 11,11,12,12-tetracyano-2-alkylanthraquinodimethane and 11,11-dicyano-12,12-bis(ethoxycarbonyl)anthraquinodimethane, anthrone derivatives such as 1-chloro-10-[bis(ethoxycarbonyl) methylene] anthrone, 1,8-dichloro-10-[bis(ethoxycarbonyl) methylene] anthrone, 1,8-dihydroxy-10-[bis(ethoxycarbonyl) methylene]anthrone, and 1-cyano-10-[bis(ethoxycarbonyl) methylene] anthrone, 7-nitro-2-aza-9-fluroenylidene-malononitrile, diphenoquinone derivatives, benzoquinone derivatives, naphtoquinone derivatives, quinine derivatives, tetracyanoethylenecyanoethylene, 2,4,8-trinitro thioxantone, dinitrobenzene derivatives, dinitroanthracene derivatives, dinitroacridine derivatives, nitroanthraquinone derivatives, dinitroanthraquinone derivatives, succinic anhydride, maleic anhydride, dibromo maleic anhydride, pyrene derivatives, carbazole derivatives, hydrazone derivatives, N,N-dialkylaniline derivatives, diphenylamine derivatives, triphenylamine derivatives, triphenylmethane derivatives, tetracyanoquinone dimethane, 2,4,5,7-tetranitro-9-fluorenone, 2,4,7-trinitro-9-dicyanomethylene fluorenone, 2,4,5,7-tetranitroxanthone derivatives, 2,4,8-trinitrothioxanthone derivatives and combinations thereof.

An electron transport compound and a UV light stabilizer can have a synergistic relationship for providing desired electron flow within the photoconductor. The presence of the UV light stabilizers alters the electron transport properties of the electron transport compounds to improve the electron transporting properties of the composite. UV light stabilizers can be ultraviolet light absorbers or ultraviolet light inhibitors that trap free radicals.

UV light absorbers can absorb ultraviolet radiation and dissipate it as heat. UV light inhibitors are thought to trap free radicals generated by the ultraviolet light and after trapping of the free radicals, subsequently to regenerate active stabilizer moieties with energy dissipation. In view of the synergistic relationship of the UV stabilizers with electron transport compounds, the particular advantages of the UV stabilizers may not be their UV stabilizing abilities, although the UV stabilizing ability may be further advantageous in reducing degradation of the organophotoreceptor over time. While not wanting to be limited by theory, the synergistic relationship contributed by the UV stabilizers may be related to the electronic properties of the compounds, which contribute to the UV stabilizing function, by further contributing to the establishment of electron conduction pathways in combination with the electron transport compounds. In particular, the organophotoreceptors with a combination of the electron transport compound and the UV stabilizer can demonstrate a more stable acceptance voltage V_{acc} with cycling. The improved synergistic performance of organophotoreceptors with layers comprising both an electron transport compound and a UV stabilizer are described further in copending U.S. Patent Application Serial Number 10/425,333 filed on April 28, 2003 to Zhu, entitled "Organophotoreceptor With A Light Stabilizer," incorporated herein by reference.

Non-limiting examples of suitable light stablizer include, for example, hindered trialkylamines such as Tinuvin 144 and Tinuvin 292 (from Ciba Specialty Chemicals, Terrytown, NY), hindered alkoxydialkylamines such as Tinuvin 123 (from Ciba Specialty Chemicals), benzotriazoles such as Tinuvan 328, Tinuvin 900 and Tinuvin 928 (from Ciba Specialty Chemicals), benzophenones such as Sanduvor 3041 (from Clariant Corp., Charlotte, N.C.), nickel compounds such as Arbestab (from Robinson Brothers Ltd, West Midlands, Great Britain), salicylates, cyanocinnamates, benzylidene malonates, benzoates, oxanilides such as Sanduvor VSU (from Clariant Corp., Charlotte, N.C.), triazines such as Cyagard UV-1164 (from Cytec Industries Inc., N.J.), polymeric sterically hindered amines such as Luchem (from Atochem North America, Buffalo, NY). In some embodiments, the light stabilizer is selected from the group consisting of hindered trialkylamines having the following formula: 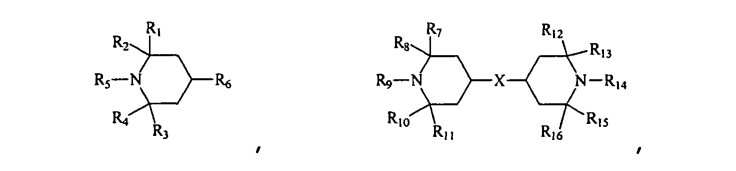 where R₁, R₂, R₃, R₄, R₆, R₇, R₈, R₁₀, R₁₁, R₁₂, R₁₃, R₁₅ and R₁₆ are, independently, hydrogen, alkyl group, or ester, or ether group; and R₅, R₉, and R₁₄ are, independently, alkyl group; and X is a linking group selected from the group consisting of -O-CO-(CH₂)ₘ-CO-O-where m is between 2 to 20.

The polymer binder for any of the particular layers of the organophotoreceptor generally is capable of dispersing or dissolving the corresponding functional compounds, such as the electron transport composition, the charge transport compound, the charge generating compound and/or the UV light stabilizing compound. Examples of suitable polymer binders generally include, for example, polystyrene-co-butadiene, polystyrene-co- acrylonitrile, modified acrylic polymers, polyvinyl acetate, styrene-alkyd resins, soya-alkyl resins, polyvinylchloride, polyvinylidene chloride, polyacrylonitrile, polycarbonates, polyacrylic acid, polyacrylates, polymethacrylates, styrene polymers, polyvinyl butyral, alkyd resins, polyamides, polyurethanes, polyesters, polysulfones, polyethers, polyketones, phenoxy resins, epoxy resins, silicone resins, polysiloxanes, poly(hydroxyether) resins, polyhydroxystyrene resins, novolak, poly(phenylglycidyl ether)-co-dicyclopentadiene, copolymers of monomers used in the above-mentioned polymers, and combinations thereof. In some embodiments of particular interest, the binder is selected from the group consisting of polycarbonates, polyvinyl butyral, and a combination thereof. Examples of suitable polycarbonate binders include, for example, polycarbonate A which is derived from bisphenol-A, polycarbonate Z, which is derived from cyclohexylidene bisphenol, polycarbonate C, which is derived from methylbisphenol A, and polyestercarbonates. Examples of suitable of polyvinyl butyral are BX-1 and BX-5 form Sekisui Chemical Co. Ltd., Japan. For a release layer, it may be desirable for the polymer to be, for example, a fluorinated polymer, siloxane polymer, fluorosilicone polymer, polysilane, polyethylene, polypropylene, polyacrylate, poly(methyl methacrylate-co-methacrylic acid), urethane resins, urethane-epoxy resins, acrylated-urethane resins, urethane-acrylic resins, crosslinked polymers thereof or a combination thereof.

Suitable optional additives for any one or more of the layers include, for example, antioxidants, coupling agents, dispersing agents, curing agents, surfactants and combinations thereof.

The photoconductive element overall typically has a thickness of from about 10 to about 45 microns. In the dual layer embodiments having a separate charge generating layer and a separate charge transport layer, charge generation layer generally has a thickness form about 0.5 to about 2 microns, and the charge transport layer has a thickness from about 5 to about 35 microns. In embodiments in which the charge transport compound and the charge generating compound are in the same layer, the layer with the charge generating compound and the charge transport composition generally has a thickness from about 7 to about 30 microns. In embodiments with a distinct electron transport layer, the electron transport layer has an average thickness from about 0.5 microns to about 10 microns and in further embodiments from about 1 micron to about 3 microns. In general, an electron transport overcoat layer can increase mechanical abrasion resistance, increases resistance to carrier liquid and atmospheric moisture, and decreases degradation of the photoreceptor by corona gases. A person of ordinary skill in the art will recognize that additional ranges of thickness within the explicit ranges above are contemplated and are within the present disclosure.

Generally, for the organophotoreceptors described herein, the charge generation compound is in an amount from about 0.5 to about 20 weight percent and in further embodiments in an amount from about 1 to about 10 weight percent, based on the weight of the photoconductive layer. The charge transport compound is in an amount from about 10 to about 80 weight percent, based on the weight of the photoconductive layer, and in further embodiments in an amount from about 35 to about 60 weight percent, based on the weight of the photoconductive layer. The electron transport compound is in an amount from about 2.5 to about 25 weight percent, based on the weight of the photoconductive layer, and in further embodiments in an amount from about 4 to about 20 weight percent, based on the weight of the photoconductive layer. The binder is in an amount from about 15 to about 80 weight percent, based on the weight of the photoconductive layer, and in further embodiments in an amount from about 20 to about 50 weight percent, based on the weight of the photoconductive layer.

For the dual layer embodiments with a separate charge generating layer and a charge transport layer, the charge generation layer generally comprises a binder in an amount from about 10 to about 90 weight percent, in further embodiments from about 15 to about 80 weight percent and in some embodiments in an amount of from about 20 to about 75 weight percent, based on the weight of the charge generation layer. The optional electron transport compound in the charge generating layer, if present, generally can be in an amount of at least about 2.5 weight percent, in further embodiments from about 4 to about 30 weight percent and in other embodiments in an amount from about 10 to about 25 weight percent, based on the weight of the charge generating layer. The charge transport layer generally comprises a binder in an amount from about 30 weight percent to about 70 weight percent. A person of ordinary skill in the art will recognize that additional ranges of binder concentrations for the dual layer embodiments within the explicit ranges above are contemplated and are within the present disclosure.

For the embodiments with a single layer having a charge generating compound and a charge transport compound, the photoconductive layer generally comprises a binder, a charge transport compound and a charge generation compound. The charge generation compound can be in an amount from about 0.05 to about 25 weight percent and in further embodiment in an amount of from about 2 to about 15 weight percent, based on the weight of the photoconductive layer. The charge transport compound can be in an amount from about 10 to about 80 weight percent, in other embodiments from about 25 to about 65 weight percent, in additional embodiments from about 30 to about 60 weight percent and in further embodiments in an amount of from about 35 to about 55 weight percent, based on the weight of the photoconductive layer, with the remainder of the photoconductive layer comprising the binder, and optionally additives, such as any conventional additives. A single layer with a charge transport composition and a charge generating compound generally comprises a binder in an amount from about 10 weight percent to about 75 weight percent, in other embodiments from about 20 weight percent to about 60 weight percent, and in further embodiments from about 25 weight percent to about 50 weight percent. Optionally, the layer with the charge generating compound and the charge transport compound may comprise an electron transport compound. The optional electron transport compound, if present, generally can be in an amount of at least about 2.5 weight percent, in further embodiments from about 4 to about 30 weight percent and in other embodiments in an amount from about 10 to about 25 weight percent, based on the weight of the photoconductive layer. A person of ordinary skill in the art will recognize that additional composition ranges within the explicit compositions ranges for the layers above are contemplated and are within the present disclosure.

In general, any layer with an electron transport layer can advantageously further include a UV light stabilizer. In particular, the electron transport layer generally can comprise an electron transport compound, a binder and an optional UV light stabilizer. An overcoat layer comprising an electron transport compound is described further in copending U.S. Patent Application Serial No. 10/396,536 to Zhu et al. entitled, "Organophotoreceptor With An Electron Transport Layer," incorporated herein by reference. For example, an electron transport compound as described above may be used in the release layer of the photoconductors described herein. The electron transport compound in an electron transport layer can be in an amount from about 10 to about 50 weight percent, and in other embodiments in an amount from about 20 to about 40 weight percent, based on the weight of the electron transport layer. A person of ordinary skill in the art will recognize that additional ranges of compositions within the explicit ranges are contemplated and are within the present disclosure.

The UV light stabilizer, if present, in any of one or more appropriate layers of the photoconductor generally is in an amount from about 0.5 to about 25 weight percent and in some embodiments in an amount from about 1 to about 10 weight percent, based on the weight of the particular layer.

The photoconductive element may be formed in accordance with any appropriate technique known in the art, such as dip coating, spray coating, extrusion and the like. A person of ordinary skill in the art will recognize that additional ranges of compositions and thickness within the explicit ranges are contemplated and are within the present disclosure.

The photoreceptor may optionally have additional layers as well. Such additional layers can be, for example, a sub-layer and/or an overcoat layer. The sub-layer can be a charge blocking layer located between the electrically conductive substrate and the photoconductive element. The sub-layer may also improve the adhesion between the electrically conductive substrate and the photoconductive element.

Overcoat layers can be, for example, barrier layers, release layers, protective layers, and adhesive layers. With respect to overcoat layers, the photoreceptor can comprise one or a plurality of overcoat layers having an electron transport composition, such as an electron transport layer. For example, the release layer or the protective layer may contain an electron transport compound. One or more of the electron transport compounds described above may be used in the release layer or the protective layer.

The electron transport compound in the release layer or the protective layer generally can be in an amount of from about 2 to about 50 weight percent and in further embodiments in an amount of from about 10 to about 40 weight percent, based on the weight of the release layer or the protective layer. A person of ordinary skill in the art will recognize that additional ranges of composition within the explicit ranges are contemplated and are within the present disclosure. While an overcoat layer may or may not have an electron transport composition, the presence of an electron transport composition in each overcoat layer (which may or may not be the same composition as in other overcoat layers) can provide continuity of electrical conductivity between a charge generating layer and the surface, which may improve the performance of the organophotoreceptor.

The release layer or the protective layer forms the uppermost layer of the photoconductor layer. A release layer is a top layer that facilitates the transfer of toner from the organophotoreceptor to an intermediate transfer medium, such as a belt or drum, or to a receiving medium, such as paper, for example, when the toner transfer is not facilitated by electrostatic forces or magnetic forces. A release layer can have a lower surface energy than the surface energy of the medium to which the toner is transferred from the organophotoreceptor. A protective layer is a top layer that provides protection for abrasion and solvent resistance to the underlayers. A layer can be both a protective layer and a release layer. The barrier layer may be sandwiched between the release layer and the photoconductive element or used to overcoat the photoconductive element. The barrier layer provides protection for abrasion and solvent resistance to the underlayers. An adhesive layer locates and improves the adhesion between a charge generating layer and an overcoat layer or between two overcoat layers.

Suitable barrier layers include, for example, coatings such as crosslinkable siloxanol-colloidal silica coating and hydroxylated silsesquioxane-colloidal silica coating, and organic binders such as polyvinyl alcohol, methyl vinyl ether/maleic anhydride copolymer, casein, polyvinyl pyrrolidone, polyacrylic acid, gelatin, starch, polyurethanes, polyimides, polyesters, polyamides, polyvinyl acetate, polyvinyl chloride, polyvinylidene chloride, polycarbonates, polyvinyl butyral, polyvinyl acetoacetal, polyvinyl formal, polyacrylonitrile, polymethyl methacrylate, polyacrylates, polyvinyl carbazoles, copolymers of monomers used in the above-mentioned polymers, vinyl chloride/vinyl acetate/vinyl alcohol terpolymers, vinyl chloride/vinyl acetate/maleic acid terpolymers, ethylene/vinyl acetate copolymers, vinyl chloride/vinylidene chloride copolymers, cellulose polymers, and mixtures thereof. The above barrier layer polymers optionally may contain small inorganic particles such as fumed silica, silica, titania, alumina, zirconia, or a combination thereof. Barrier layers are described further in U.S. Patent 6,001,522 to Woo et al., entitled Barrier Layer For Photoconductor Elements Comprising An Organic Polymer And Silica," incorporated herein by reference.

The release layer topcoat may comprise, for example, any release layer composition known in the art. In some embodiments, the release layer is a fluorinated polymer, siloxane polymer, fluorosilicone polymer, polysilane, polyethylene, polypropylene, polyacrylate, poly(methyl methacrylate-co-methacrylic acid), urethane resins, urethane-epoxy resins, acrylated-urethane resins, urethane-acrylic resins, or a combination thereof. The release layers can comprise crosslinked polymers.

The protective layer protects the organophotoreceptor from chemical and mechanical degradation. The protective layer may comprise, for example, any protective layer composition known in the art. In some embodiments, the protective layer is a fluorinated polymer, siloxane polymer, fluorosilicone polymer, polysilane, polyethylene, polypropylene, polyacrylate, poly(methyl methacrylate-co-methacrylic acid), urethane resins, urethane-epoxy resins, acrylated-urethane resins, urethane-acrylic resins, or a combination thereof. In some embodiments, the protective layer comprises crosslinked polymers.

Generally, adhesive layers comprise a film forming polymer, such as polyester, polyvinylbutyral, polyvinylpyrolidone, polyurethane, polymethyl methacrylate, poly(hydroxy amino ether) and the like. Overcoat layers are described further in U.S. Patent 6,180,305 to Ackley et al., entitled "Organic Photoreceptors For Liquid Electrophotography," incorporated herein by reference.

Sub-layers can comprise, for example, polyvinylbutyral, organosilanes, hydrolyzable silanes, epoxy resins, polyesters, polyamides, polyurethanes, silicones and the like. In some embodiments, the sub-layer has a dry thickness between about 20 Angstroms and about 2,000 Angstroms. Sublayers containing metal oxide conductive particles can be 1-25 microns thick. A person of ordinary skill in the art will recognize that additional ranges of compositions and thickness within the explicit ranges are contemplated and are within the present disclosure.

The organophotoreceptors as described herein are suitable for use in an imaging process with either dry or liquid toner development including, for example, dry toners and liquid toners known in the art. Liquid toner development can be desirable because it offers the advantages of providing higher resolution images. Examples of suitable liquid toners are known in the art. Liquid toners generally comprise toner particles dispersed in a carrier liquid. The toner particles generally can comprise a colorant/pigment, a resin binder, and/or a charge director. In some embodiments of liquid toner, a resin to pigment ratio can be from 2:1 to 10:1, and in other embodiments, from 4:1 to 8:1. Liquid toners are described further in Published U.S. Patent. Applications 2002/0128349, entitled "Liquid Inks Comprising A Stable Organosol," 2002/0086916, entitled "Liquid Inks Comprising Treated Colorant Particles," and 2002/0197552, entitled "Phase Change Developer For Liquid Electrophotography," all three of which are incorporated herein by reference.

### Toluidine-Based Charge Transport Materials

Organophotoreceptors described herein comprise an electron transport compound having the formula: 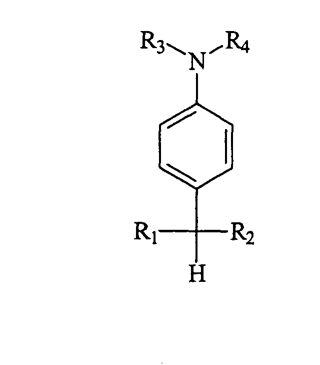 where R₁ and R₂ are, independently, an (N,N-disubstituted) aminoaryl group (such as a triphenylamine group), a carbazolyl group, or a julolidine group, and R₃ and R₄ are, independently, hydrogen, branched or linear alkyl group (e.g., a C₁-C₂₀ alkyl group) , branched or linear unsaturated hydrocarbon group, cycloalkyl group (e.g. cyclohexyl group), or aryl group (e.g., phenyl group, naphthyl group, stilbenyl group, (9H-fluoren-9-ylidene)benzyl group, or tolanyl group).

Specific, non-limiting examples of suitable charge transport materials within the general formula of the toluidine-based compounds have the following structures.

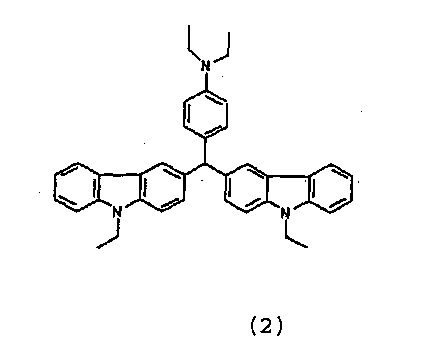

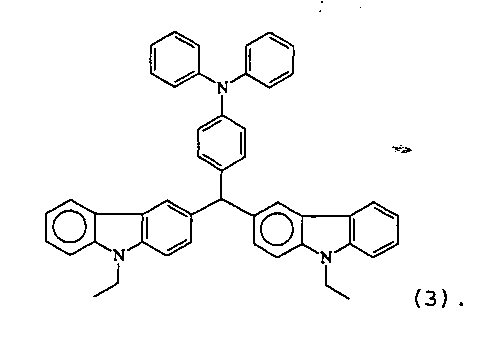

The toluidine-based compounds can be synthesized, for example, from an N,N-disubstituted aminobenzaldehyde and a corresponding aromatic adduct. For example, compounds 2 and 3 can respectively be synthesized from p-diethylaminobenzaldehyde or p-diphenylaminobenzaldehyde, respectively, with N-ethylcarbazole (all three of which are available from Aldrich, Milwaukee, WI.). Other N,N-disubstituted aminobenzaldehydes are also commercially available. Other aromatic adducts, such as triphenyl amine and julolidine are also commercially available from Aldrich (Milwaukee, WI). The synthesis of compound 2 is described in detail below. Other toluidine-based compounds can be similarly synthesized based on the description herein.

### Organophotoreceptor Properties With Toluidine-Based Compounds

As demonstrated in the examples below, organophotoreceptors with toluidine-based compounds can have improved cycling properties in electrostatic testing. Using the testing procedure described below, the improved organophotoreceptors can have V_{acc} values after 4000 cycles of at least about 500 volts, in further embodiments at least about 600 volts and in other embodiments at least about 675 volts. Furthermore, the organophotoreceptors can have contrast voltages (V_{con}) after 4000 cycles of at least about 425 volts, in further embodiments at least about 450 volts, and in other emboidments at least about 475 volts. A person of ordinary skill in the art will recognize that additional values of electrostatic properties within these explicit values are contemplated and are within the present disclosure.

### Organophotoreceptor (OPR) Preparation Methods

Conveniently, the photoconductive element may be formed by dispersing or dissolving the component compounds, such as a charge generating compound, a charge transport compound, a light stabilizer, an electron transport compound, and/or a polymeric binder in an organic solvent, coating the dispersion and/or solution on the respective underlying layer and drying the coating. In some embodiments, the components can be dispersed by high shear homogenization, ball-milling, attritor milling, high energy bead (sand) milling or other size reduction processes or mixing approaches known in the art for effecting particle size reduction in forming a dispersion. The coatings can be applied, for example, using knife coating, extrusion, dip coating or other appropriate coating approaches, including those known in the art. In some embodiments, a plurality of layers are applied as sequential coatings. The layers can be dried prior to the application of a subsequent layer. Some specific examples are presented below.

The invention will now be described further by way of the following illustrative and non-limiting examples. These examples are to be viewed as being illustrative of specific materials falling within the broader disclosure presented above and are not to be viewed as limiting the broader disclosure in any way.

### EXAMPLES

### Example 1 - Synthesis of Electron Transport Compounds

This example describes the synthesis of two electron transport compounds. One compound has a toluidine group, compound 2 above. The second compound is used for the formation of comparative organophotoreceptors.

### Preparation of Compound 2

A 20 g quantity of N-ethylcarbazole (0.1 mole, commercially obtained from Aldrich, Milwaukee, WI), 7.4 g of p-diethylaminobenzaldehyde (0.042 mole, commercially obtained from Aldrich, Milwaukee, WI), 40 ml of isopropyl alcohol were added to a 100 m 3-neck flask equipped with reflux condenser and mechanical stirrer. A 7 ml quantity of concentrated sulfuric acid was added to this solution dropwise at a rate of - 1 ml per 2 min. After the addition of the acid was completed, urea (5 g, 0.08 mole, commercially obtained from Aldrich, Milwaukee, WI) was added. The mixture was refluxed for 4 hours, cooled to room temperature and then added slowly with stirring to a 2 liter flask containing 1500 g of 1% sodium hydrogen bicarbonate in ice water. Stirring was continued for an additional 2 hours. Then, the solid was filtered off and washed repeatedly with water until the pH of the washing water was neutral. The product was recrystalized 3 times from a mixture of acetone and methanol to obtain 11g of a light blue solid at a 48% yield. The solid was found to have a melting point of 152°C. The proton NMR spectrum (H-NMR) was obtained in CDCl₃. The peaks were found at (ppm) : 1.01 - 1.24 (t , 6H) ; 1.30 - 1.56 (t , 6H); 3.19 - 3.42 (q , 4H) ; 4.19 - 4.43 (q , 4H) ; 6.53 (s, 1H); 6.94 - 7.52 (m , 14H) ; 7.75 - 8.05 (m , 4H).

### Preparation of (4-n-Butoxycarbonyl-9-fluorenylidene) Malononitrile

A 460 g quantity of concentrated sulfuric acid (4.7 moles, analytical grade, commercially obtained from Sigma-Aldrich, Milwaukee, WI) and 100 g of diphenic acid (0.41 mole, commercially obtained from Acros Fisher Scientific Company Inc., Hanover Park, IL) were added to a 1-liter 3-neck round bottom flask, equipped with a thermometer, mechanical stirrer and a reflux condenser. Using a heating mantle, the flask was heated to 135-145 °C for 12 minutes, and then cooled to room temperature. After cooling to room temperature, the solution was added to a 4-liter Erlenmeyer flask containing 3 liter of water. The mixture was stirred mechanically and was boiled gently for one hour. A yellow solid was filtered out hot, washed with hot water until the pH of the wash-water was neutral, and was air-dried overnight. The yellow solid was fluorenone-4-carboxylic acid. The yield was 75 g (80%). The product was then characterized. The melting point (m.p.) was found to be 223-224 °C. A ¹H-NMR spectrum of fluorenone-4-carboxylic acid was obtained in d₆-DMSO solvent with a 300 MHz NMR from Bruker Instrument. The peaks were found at (ppm) δ = 7.39-7.50 (m, 2H); δ = 7.59 - 7.70 (q, 2H); δ = 7.74 - 7.85 (d, 1H); δ = 7.88 -8.00 (d, 1H ); and δ = 8.18 - 8.30 (d, 1H), where d is doublet, t is triplet, m is multiplet, dd is double doublet, q is quintet.

A 70 g (0.312 mole) quantity of fluorenone-4-carboxylic acid, 480 g (6.5 mole) of n-butanol (commercially obtained from Fisher Scientific Company Inc., Hanover Park, IL), 1000 ml of toluene and 4 ml of concentrated sulfuric acid were added to a 2-liter round bottom flask equipped with a mechanical stirrer and a reflux condenser with a Dean Stark apparatus. The solution was refluxed for 5 hours with aggressive agitation and refluxing, during which time - 6 g of water were collected in the Dean Stark apparatus. The flask was cooled to room temperature. The solvents were evaporated, and the residue was added, with agitation, to 4-liter of a 3% sodium bicarbonate aqueous solution. The solid was filtered off, washed with water until the pH of the wash-water was neutral, and dried in a hood overnight. The product was n-butyl fluorenone-4-carboxylate ester. The yield was 70 g (80%). A ¹H-NMR spectrum of n-butyl fluorenone-4-carboxylate ester was obtained in CDCl₃ with a 300 MHz NMR from Bruker Instrument. The peaks were found at (ppm) δ = 0.87 -1.09 (t, 3H); δ = 1.42 - 1.70 (m, 2H); δ = 1.75 - 1.88 (q, 2H); δ = 4.26 -4.64 (t, 2H); δ = 7.29 -7.45 (m, 2H); δ = 7.46 -7.58 (m, 1H); δ = 7.60 - 7.68 (dd, 1H); δ = 7.75 - 7.82 (dd, 1H); δ = 7.90 -8.00 (dd, 1H); δ = 8.25 - 8.35 (dd, 1H).

A 70 g (0.25 mole) quantity of n-butyl fluorenone-4-carboxylate ester, 750 ml of absolute methanol, 37 g (0.55 mole) of malononitrile (commercially obtained from Sigma-Aldrich, Milwaukee, WI), 20 drops of piperidine (commercially obtained from Sigma-Aldrich, Milwaukee, WI) were added to a 2-liter, 3-neck round bottom flask equipped with a mechanical stirrer and a reflux condenser. The solution was refluxed for 8 hours. Then, the flask was cooled to room temperature. The orange crude product was filtered, washed twice with 70 ml of methanol and once with 150 ml of water, and dried overnight in a hood. The orange crude product was recrystalized from a mixture of 600 ml of acetone and 300 ml of methanol using activated charcoal. The flask was placed at 0 °C for 16 hours. The crystals were filtered and dried in a vacuum oven at 50 °C for 6 hours to obtain 60 g of pure (4-n-butoxycarbonyl-9-fluorenylidene) malononitrile. The melting point (m.p.) of the solid was found to be 99-100 °C. A ¹H-NMR spectrum of (4-n-butoxycarbonyl-9-fluorenylidene) malononitrile was obtained in CDCl₃ with a 300 MHz NMR from Bruker Instrument. The peaks were found at (ppm) δ = 0.74 - 1.16 (t, 3H); δ = 1.38 - 1.72 (m, 2H); δ = 1.70 -1.90 (q, 2H); δ = 4.29 - 4.55 (t, 2H); δ = 7.31 - 7.43 (m, 2H); δ = 7.45 - 7.58 (m, 1H); δ = 7.81 - 7.91 (dd, 1H); δ = 8.15 - 8.25 (dd, 1H); δ = 8.42 - 8.52 (dd, 1H ); δ = 8.56 - 8.66 (dd, 1H) .

### Example 2 - Preparation of Organophotoreceptors

The preparation of three organophotoreceptor samples and three corresponding comparative organophotoreceptor samples are described in this example. The samples are characterized in the following example.

### Sample 1

Sample 1 was a single layer organophotoreceptor having a 76.2 micron (3 mil) thick polyester substrate with a layer of vapor-coated aluminum (commercially obtained from CP Films, Martinsville, VA). The coating solution for the single layer organophotoreceptor was prepared by pre-mixing 2.4 g of a 20 weight % solution of Compound 2 in tetrahydrofuran, 6.66 g of a 25 weight % solution of MPCT-10 (a charge transfer material, commercially obtained from Mitsubishi Paper Mills, Tokyo, Japan) in tetrahydrofuran, 7.65 g of a 12 weight % solution of polyvinyl butyral resin (BX-1, commercially obtained from Sekisui Chemical Co. Ltd., Japan) in tetrahydrofuran. A 0.74 g quantity of a CGM mill-base containing 19 weight % of titanyl oxyphthalocyanine and a polyvinyl butyral resin (BX-5, commercially obtained from Sekisui Chemical Co. Ltd., Japan) at a weight ratio of 2.3:1 was then added to the above mixture. The CGM mill-base was prepared by milling 112.7 g of titanyl oxyphthalocyanine (commercially obtained from H.W. Sands Corp., Jupiter, FL) with 49 g of the polyvinyl butyral resin (BX-5) in 651 g of methylethylketone on a horizontal sand mill (model LMC12 DCMS, commercially obtained from Netzsch Incorporated, Exton, PA) with 1-micron zirconium beads using recycle mode for 4 hours. After mixing on a mechanical shaker for about 1 hour, a single layer coating solution was coated onto the substrate described above using a knife coater with a gap spacing of 94 microns. The substrate with the coating solution was dried in an oven at 110°C for 5 minutes. The dry layer thickness was 9 microns.

### Sample 2

Sample 2 was prepared as described for Sample 1 except that, after mixing on a mechanical shaker for about 1 hour, the single layer coating solution was coated onto the substrate described above using a knife coater with a gap spacing of 145 microns. The substrate with the coating solution was dried in an oven at 110°C for 5 minutes. The dry layer thickness was 14 microns.

### Sample 3

Sample 3 was prepared as described for Sample 1 except that, after mixing on a mechanical shaker for about 1 hour, the single layer coating solution was coated onto the substrate described above using a knife coater with a gap space of 200 microns. The substrate with the coating solution was dried in an oven at 110°C for 5 minutes. The dry layer thickness was 19 microns.

### Comparative Sample A

Comparative Sample A was a single layer organophotoreceptor having a 76.2 micron (3 mil) thick polyester substrate having a layer of vapor-coated aluminum (commercially obtained from CP Films, Martinsville, VA). The coating solution for the single layer organophotoreceptor was prepared by pre-mixing 2.4 g of a 20 weight % solution (4-n-butoxycarbonyl-9-fluorenylidene) malononitrile in tetrahydrofuran, 6.66 g of 25 weight % MPCT-10 (a charge transfer material, commercially obtained from Mitsubishi Paper Mills, Tokyo, Japan) solution in tetrahydrofuran, 7.65 g of 12 weight % polyvinyl butyral resin (BX-1, commercially obtained from Sekisui Chemical Co. Ltd., Japan) in tetrahydrofuran. A 0.74 g quantity of a CGM mill-base containing 19 weight % of titanyl oxyphthalocyanine and a polyvinyl butyral resin (BX-5, commercially obtained from Sekisui Chemical Co. Ltd., Japan) at a weight ratio of 2.3:1 was then added to the above mixture. The CGM mill-base was prepared by milling 112.7 g of titanyl oxyphthalocyanine (commercially obtained from H.W. Sands Corp., Jupiter, FL) with 49 g of the polyvinyl butyral resin (BX-5) in 651 g of methylethylketone on a horizontal sand mill (model LMC12 DCMS, commercially obtained from Netzsch Incorporated, Exton, PA) with 1-micron zirconium beads using recycle mode for 4 hours. After mixing on a mechanical shaker for about 1 hour, a single layer coating solution was coated onto the substrate described above using a knife coater with a gap spacing of 94 microns. The substrate with the coating solution was dried in an oven at 110°C for 5 minutes. The dry layer thickness was 10 microns.

### Comparative Sample B

Comparative Sample B was prepared as described for Comparative Sample A except that, after mixing on a mechanical shaker for about 1 hour, the single layer coating solution was coated onto the substrate described above using a knife coater with a gap space of 145 microns. The substrate with the coating solution was dried in an oven at 110°C for 5 minutes.- The dry layer thickness was 15 microns.

### Comparative Sample C

Comparative Sample C was prepared as described for Comparative Sample A except that, after mixing on a mechanical shaker for about 1 hour, the single layer coating solution was coated onto the substrate described above using a knife coater with a gap space of 200 microns. The substrate with the coating solution was dried in an oven at 110°C for 5 minutes. The dry layer thickness was 20 microns.

### Example 3 - Electrostatic Testing And Properties Of Organophotoreceptors

This example provides results of electrostatic testing on the organophotoreceptor samples formed as described in Example 2.

Electrostatic cycling performance of organophotoreceptors described herein with toluidine-based compounds was determined using in-house designed and developed test bed that can test, for example, up to three sample strips wrapped around a 160 mm diameter drum. The results on these samples are indicative of results that would be obtained with other support structures, such as belts, drums and the like, for supporting the organophotoreceptors.

For testing using a 160 mm diameter drum, three coated sample strips, each measuring 50 cm long by 8.8 cm wide, are fastened side-by-side and completely around an aluminum drum (50.3 cm circumference). In some embodiments, at least one of the strips is a control sample that is precision web coated and used as an internal reference point. A comparative sample was used as the control. In this electrostatic cycling tester, the drum rotated at a rate of 8.13 cm /sec (3.2ips), and the location of each station in the tester (distance and elapsed time per cycle) is given as shown in the following table:

**Table 1**

| Electrostatic test stations around the 160 mm diameter drum at 8.13 cm /sec. | | | |
|---|---|---|---|
| **Station** | **Degrees** | **Total Distance, cm** | **Total Time, sec** |
| Front erase bar edge | 0° | Initial, 0 cm | Initial, 0 s |
| Erase Bar | 0 - 7.2° | 0 - 1.0 | 0 - 0.12 |
| Scorotron Charger | 113.1 - 135.3° | 15.8 - 18.9 | 1.94 - 2.33 |
| Laser Strike | 161.0° | 22.5 | 2.77 |
| Probe #1 | 181.1° | 25.3 | 3.11 |
| Probe #2 | 251.2° | 35.1 | 4.32 |
| Erase bar | 360° | 50.3 | 6.19 |

The erase bar is an array of laser emitting diodes (LED) with a wavelength of 720 nm. that discharges the surface of the organophotoreceptor. The scorotron charger comprises a wire that permits the transfer of a desired amount of charge to the surface of the organophotoreceptor.

From the above table, the first electrostatic probe (Trek 344^{TM} electrostatic meter, Trek, Inc. Medina, NY) is located 0.34 s after the laser strike station and 0.78 s after the scorotron while the second probe (Trek^{TM} 344 electrostatic meter) is located 1.21 s from the first probe and 1.99 s from the scorotron. All measurements are performed at ambient temperature and relative humidity.

Electrostatic measurements were obtained as a compilation of several runs on the test station. The first three diagnostic tests (prodtest initial, VlogE initial, dark decay initial) were designed to evaluate the electrostatic cycling of a new, fresh sample and the last three, identical diagnostic test (prodtest final, VlogE final, dark decay final) are run after cycling of the sample. In addition, measurements were made periodically during the test, as described under "longrun" below. The laser is operated at 780nm wavelength, 600dpi, 50 micron spot size, 60 nanoseconds / pixel expose time, 1,800 lines per second scan speed, and a 100% duty cycle. The duty cycle is the percent exposure of the pixel clock period, i.e., the laser is on for the full 60 nanoseconds per pixel at a 100% duty cycle.

### Electrostatic Test Suite:

1) PRODTEST: Charge acceptance (V_{acc}) and discharge voltage (V_{dis}) were established by subjecting the samples to corona charging (erase bar always on) for three complete drum revolutions (laser off); discharged with the laser @ 780nm & 600dpi on the forth revolution (50 um spot size, expose 60 nanoseconds / pixel, run at a scan speed of 1,800 lines per second, and use a 100% duty cycle); completely charged for the next three revolutions (laser off); discharged with only the erase lamp @ 720nm on the eighth revolution (corona and laser off) to obtain residual voltage (Vᵣₑₛ); and, finally, completely charged for the last three revolutions (laser off). The contrast voltage (V_{con}) is the difference between V_{acc} and V_{dis} and the functional dark decay (V_{dd}) is the difference in charge acceptance potential measured by probes #1 and #2.
2) VLOGE: This test measures the photoinduced discharge of the photoconductor to various laser intensity levels by monitoring the discharge voltage of the sample as a function of the laser power (exposure duration of 50 ns) with fixed exposure times and constant initial potentials. The functional photosensitivity, S₇₈₀ₙₘ, and operational power settings was determined from this diagnostic test.
3) DARK DECAY: This test measures the loss of charge acceptance in the dark with time without laser or erase illumination for 90 seconds and can be used as an indicator of i) the injection of residual holes from the charge generation layer to the charge transport layer, ii) the thermal liberation of trapped charges, and iii) the injection of charge from the surface or aluminum ground plane.
4) LONGRUN: The sample was electrostatically cycled for 4,000 drum revolutions according to the following sequence per each sample-drum revolution. The sample was charged by the corona, the laser was cycled on and off (80-100° sections) to discharge a portion of the sample and, finally, the erase lamp discharged the whole sample in preparation for the next cycle. The laser was cycled so that the first section of the sample was never exposed, the second section was always exposed, the third section was never exposed, and the final section was always exposed. This pattern was repeated for a total of 4,000 drum revolutions, and the data was recorded periodically, after every 50th cycle for the 4,000 cycle longrun.
5) After the LONGRUN test, the PRODTEST, VLOGE, DARK DECAY diagnostic tests were run again.

The following Table shows the results from the prodtest initial and prodtest final diagnostic tests. The values for the charge acceptance voltage (V_{acc}, probe #1 average voltage obtained from the third cycle), discharge voltage (V_{dis}, probe #1 average voltage obtained from the fourth cycle) are reported for the initial and final cycles.

**Table 2.**

| Dry Electrostatic Test Results after 4000 cycles. | | | | | | | | | | | |
|---|---|---|---|---|---|---|---|---|---|---|---|
| Sample | Prodtest Initial | | | | | | Prodtest Final | | | | |
| | V_{acc} | V_{dis} | V_{con} | S₇₈₀ₙₘ | Dark Decay | Vᵣₑₛ | V_{acc} | V_{dis} | V_{con} | Dark Decay | Vᵣₑₛ |
| Sample 1 | 418 | 61 | 357 | 322 | 60 | 30 | 512 | 65 | 447 | 84 | 19 |
| Sample 2 | 457 | 59 | 398 | 330 | 87 | 24 | 564 | 92 | 472 | 94 | 26 |
| Sample 3 | 522 | 90 | 432 | 210 | 96 | 29 | 685 | 193 | 492 | 63 | 67 |
| Comparative Sample A | 605 | 37 | 568 | 356 | 38 | 12 | 326 | 34 | 292 | 82 | 13 |
| Comparative Sample B | 716 | 32 | 684 | 424 | 34 | 10 | 319 | 30 | 289 | 127 | 8 |
| Comparative Sample C | 730 | 57 | 673 | 430 | 42 | 18 | 450 | 47 | 403 | 148 | 11 |
| Note: The data were obtained on a sample at the beginning of cycling and after 4,000 charge-discharge cycles. | | | | | | | | | | | |

In the above table, the radiation sensitivity (Sensitivity at 780nm in m²/J) of the xerographic process was determined from the information obtained during the VLOGE diagnostic run by calculating the reciprocal of the product of the laser power required to discharge the photoreceptor to ½ of its initial potential, the exposure duration, and 1/spot size.

The results in Table 2 indicate that compound 2 improves the performance of the organophotoreceptor relative to organophotoreceptors formed with a comparative electron transport compound. Specifically, the organophotoreceptor samples exhibit an increase in acceptance voltage (V_{acc}) compared with a significant decrease in acceptance voltage for the comparative samples. Thus, while the discharge voltage is higher for the samples relative to the comparative examples, the contrast voltage (V_{con}) is higher for the samples after 4000 cycles compared with the comparative samples.

As understood by those skilled in the art, additional substitution, variation among substituents, and alternative methods of synthesis and use may be practiced within the scope and intent of the present disclosure of the invention. The embodiments above are intended to be illustrative and not limiting. Additional embodiments are within the claims. Although the present invention has been described with reference to particular embodiments, workers skilled in the art will recognize that changes may be made in form and detail without departing from the spirit and scope of the invention.

Attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

## Claims

1. An organophotoreceptor comprising an electrically conductive substrate and a photoconductive element on said electrically conductive substrate wherein said photoconductive element comprises
(a) a charge transport material having the formula 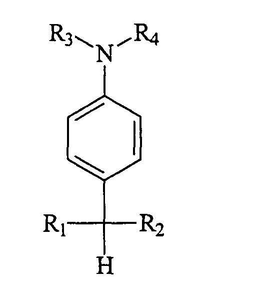 where R₁ and R₂ are, independently, an (N,N-disubstituted) aminoaryl group, a carbazolyl group, or a julolidine group, and R₃ and R₄ are, independently, hydrogen, branched or linear alkyl group, branched or linear unsaturated hydrocarbon group, cycloalkyl group, or aryl group; and
(b) a charge generating compound.

2. An organophotoreceptor according to claim 1 wherein said photoconductive layer further comprises a binder.

3. An organophotoreceptor according to either one of claims 1 and 2 wherein said charge transport material has the formula 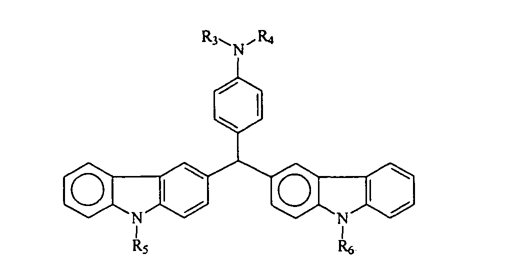 wherein R₃, R₄, R₅ and R₆ are, independently, hydrogen, branched or linear alkyl group, branched or linear unsaturated hydrocarbon group, cycloalkyl group, or aryl group.

4. An organophotoreceptor according to any preceding claim further comprises an overcoat layer on said photoconductive element.

5. An organophotoreceptor according to any preceding claim wherein said photoconductive element further comprises a UV stabilizer.

6. An organophotoreceptor according to any preceding claim wherein said charge generating compound and said charge transport material are in one layer of said photoconductive element.

7. An organophotoreceptor according to any preceding claim wherein said photoconductive element comprises a first layer and a second layer with said first layer comprising said charge generating compound and said second layer comprising said charge transport material.

8. An organophotoreceptor according to any preceding claim further comprising a charge transport compound, wherein said charge generating compound, said charge transport material and said charge transport compound are in one layer of said photoconductive element.

9. An organophotoreceptor according to any one of claims 1 to 7 further comprising a charge transport compound wherein said photoconductive element comprising a first layer and a second layer with said first layer comprising said charge generating compound and said charge transport compound and said second layer comprising said charge transport material.

10. An organophotoreceptor according to any one of claims 1 to 7 further comprising a charge transport compound wherein said photoconductive element comprises a first layer, a second layer and a third layer with said first layer comprising said charge generating compound, said second layer comprising said charge transport compound and said third layer comprising said charge transport material.

11. An organophotoreceptor according to any preceding claim wherein R₁ and R₂ are a carbazolyl group.

12. An organophotoreceptor according to any preceding claim wherein the (N,N-disubstituted) aminoaryl group is a triphenyl amine group.

13. An organophotorecpetor according to any preceding claim having a contrast voltage (V_{con}) after 4000 test cycles of at least about 425 volts.

14. An electrophotographic imaging apparatus comprising:
(a) a light imaging component; and
(b) an organophotoreceptor oriented to receive light from the light imaging component, the organophotoreceptor comprising an electrically conductive substrate and a photoconductive element on said electrically conductive substrate wherein said photoconductive element comprises:
(i) a charge transport material having the formula 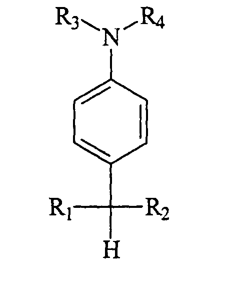 where R₁ and R₂ are, independently, a carbazolyl group, an (N,N-disubstituted) aminoaryl group, or a julolidine group, and R₃ and R₄ are, independently, hydrogen, branched or linear alkyl group, branched or linear unsaturated hydrocarbon group, cycloalkyl group, or aryl group; and
(ii) a charge generating compound.

15. An electrophotographic imaging apparatus according to claim 14 wherein said charge transport material having the formula 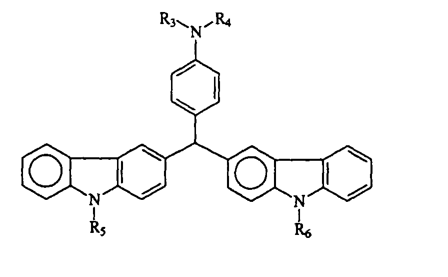 where R₃, R₄, R₅ and R₆ are, independently, hydrogen, branched or linear alkyl group, branched or linear unsaturated hydrocarbon group, cycloalkyl group, or aryl group.

16. An electrophotographic imaging apparatus according to either one of claims 14 and 15 further comprises an overcoat layer on said photoconductive element.

17. An electrophotographic imaging apparatus according to any one of claims 14 to 16 wherein said photoconductive element further comprises a UV stabilizer.

18. An electrophotographic imaging apparatus according to any one of claims 14 to 17 wherein said photoconductive element further comprises a charge transport compound.

19. An electrophotographic imaging process comprising:
(a) applying an electrical charge to a surface of an organophotoreceptor comprising an electrically conductive substrate and a photoconductive element on said electrically conductive substrate wherein said photoconductive element comprises:
(i) a charge transport material having the formula 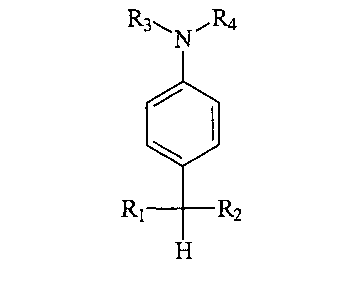 where R₁ and R₂ are, independently, a carbazolyl group, an (N,N-disubstituted) aminoaryl group, or a julolidine group, and R₃ and R₄ are, independently, hydrogen, branched or linear alkyl group, branched or linear unsaturated hydrocarbon group, cycloalkyl group, or aryl group; and
(ii) a charge generating compound;
(b) imagewise exposing said surface of said organophotoreceptor to radiation to dissipate charge in selected areas and thereby form a pattern of charged and uncharged areas on said surface;
(c) contacting said surface with a toner to create a toned image; and
(d) transferring said toned image to a substrate.

20. An electrophotographic imaging process according to claim 19 wherein said toner comprises a liquid toner comprising a dispersion of colorant particles in an organic liquid.

21. An electrophotographic imaging process according to either one of claims 19 and 20 wherein said charge transport material has the formula 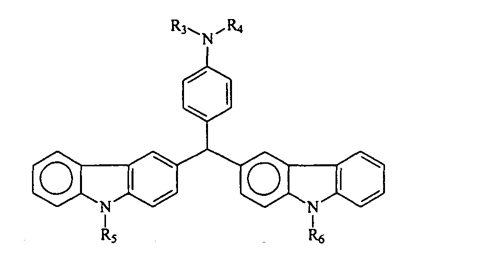 where R₃, R₄, R₅ and R₆ are, independently, hydrogen, branched or linear alkyl group, branched or linear unsaturated hydrocarbon group, cycloalkyl group, or aryl group.

22. An electrophotographic imaging process according to any one of claims 19 to 21 further comprises an overcoat layer on said photoconductive element.

23. An electrophotographic imaging process according to any one of claims 19 to 22 wherein said photoconductive element further comprises a light stabilizer.

24. An electrophotographic imaging process according to any one of claims 19 to 23 wherein said photoconductive layer comprises a charge transport compound.

25. A charge transport material having the formula 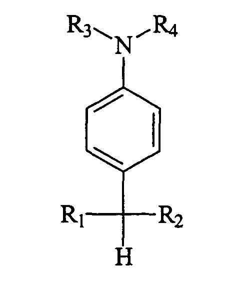 where R₁ and R₂ are, independently, a carbazolyl group, an (N,N-disubstituted) aminoaryl group, or a julolidine group, and R₃ and R₄ are, independently, hydrogen, branched or linear alkyl group, branched or linear unsaturated hydrocarbon group, cycloalkyl group, or aryl group.

26. A charge transport material according to claim 25 wherein said charge transport material has the formula 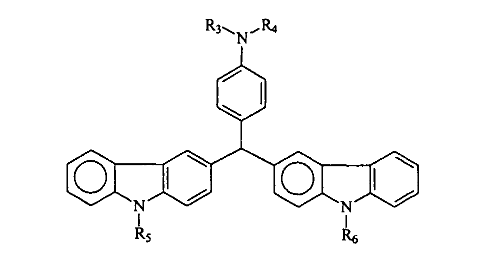 where R₃, R₄, R₅ and R₆ are, independently, hydrogen, branched or linear alkyl group, branched or linear unsaturated hydrocarbon group, cycloalkyl group, or aryl group.

27. A charge transport material according to claim 25 wherein R₁ and R₂ are a carbazolyl group.

28. A charge transport material according to claim 25 wherein said (N,N-disubstituted) aminoaryl group is a triphenyl amine group.
